# EUROPEAN PATENT SPECIFICATION

(11) **EP 2 114 578 B1**
(45) Date of publication and mention of the grant of the patent: **01.08.2012**
(21) Application number: 07848639.6
(22) Date of filing: 20.12.2007
(51) Int. Cl.: B05B 1/34, A61L 2/22, B08B 3/02

(54) **SPRAY DEVICE**
SPRÜHVORRICHTUNG
DISPOSITIF DE PULVÉRISATION

(30) Priority: 21.12.2006 GB 0625687
(43) Date of publication of application: 11.11.2009
(73) Proprietor: Hughes Safety Showers Limited, Bredbury Stockport Cheshire SK6 2SS (GB)
(72) Inventor: GHAVAMI-NASR, Ghasem, Manchester M6 6AP (GB); YULE, Andrew John, Manchester M6 6AP (GB)
(74) Representative: Hickinson, Katie Louise
(86) International application number: PCT/GB2007/004910
(87) International publication number: WO 2008/075063

(56) References cited:
- DE-A1- 3 235 080
- GB-A- 2 017 534
- US-A- 4 186 877
- US-A- 5 775 588

## Description

The present invention relates to spray devices for generating a spray or aerosol of fine droplets, in particular for coating objects, personnel, clothing etc., for example, with a decontamination liquid and for fire supression.

Fine sprays, which are defined herein as sprays with a mean droplet size of less than 80 microns, can act to more efficiently coat an object without wetting it significantly. Coarser sprays, for example those produced by hand-held pumps or showers, that attempt to wash off materials act in a different way from the present invention to decontaminate objects. Such sprays may be used for antibacterial, disinfectant, decontamination or other hygiene purposes. Fine sprays are also useful for cleaning particulates, for example smoke, from the atmosphere, and also for cooling the local atmosphere by droplet evaporation. Such fine sprays can also be used for the suppression of fires.

Existing fine spray decontamination devices generally use either compressed air assistance to produce fine sprays or use hydraulic swirl atomisers. The former is undesirable due to the need to provide a source of compressed air. The latter is undesirable because to reduce the drop size generated by a swirl atomiser, either the supply pressure of the liquid to the swirl atomiser must be increased, resulting in liquid flow rates which are too high, or the size of the orifice through which the spray is generated must be reduced to sizes of around 0.1mm, which is unfeasible due to the likelihood of erosion, blockage and manufacturing difficulties. High liquid flow rates result in an undesirable wetting of the surface which is to be decontaminated and in the use of high volumes of decontamination liquid which leads to problems of disposal of used decontamination liquid. Other methods use ultrasonic, electrical or electro-static atomisers to generate a fine spray, however these tend to result in complicated and relatively expensive devices which require close manufacturing tolerances and which are difficult to maintain.

Document DE 3235 080 discloses a spray device according with the preamble of claim 1.

These problems are avoided by the use of spill-return swirl atomisers, which bleed liquid from inside the atomiser, back to the pump reservoir, and so allow the use of high pressure so as to generate the desired small droplets (less than 80 microns and preferably less than 30 microns), but with sufficiently low flow rates (less than 0.3 litres per minute and preferably less than 0.1 litres per minute).

In accordance with the present invention there is provided a spray device as set out in claim 1. Preferred embodiments of the present invention are laid down in the appended dependent claims 2-20.

According to the present invention there is provided a spray device comprising at least one swirl atomiser for generating a spray of a liquid and a source of pressurised liquid for feeding the or each swirl atomiser, wherein each swirl atomiser comprises:
a housing defining a tubular chamber having a side wall and two opposing end walls;
at least one inlet channel from the source oriented in the side wall so as to generate a vortex of liquid within the chamber;
a bleed outlet channel in a second of the end walls for bleeding any excess liquid from the atomiser; and
a spray outlet channel in a first of the end walls, which is narrower than the bleed outlet channel;
and wherein the length of the chamber between the end walls is at least twice the width of the chamber between opposing faces of the side wall. The action of the bleed channel reduces the rate at which liquid is dispensed from the atomiser, while not disrupting the formation of the fine spray. This arrangement results in a very simple spray device, which is easily re-filled and maintained, is reliable and relatively cheap to manufacture. The source of pressurised liquid may comprise a reservoir of liquid and a pump for pumping liquid from the reservoir to the, or each atomiser.

Such a spray device is very useful as a decontamination device, in which case, the liquid is a decontamination liquid. Alternatively, the device can be used for spray cleaning or cooling of surfaces or of gas, e.g. of warm atmospheric air. The spray device provides good spray penetration, as well as a narrow spray angle.

In order not to waste the liquid which is bled from the atomiser, the device preferably includes a recirculation system for recirculating the excess liquid to the source. Where the source of pressurised liquid comprises a reservoir of liquid and a pump, it is preferred that the recirculation system comprises a channel extending from the, or each atomiser to the reservoir.

Each swirl atomiser comprises a housing defining a tubular chamber having a side wall, preferably a continuously curved side wall, and two opposing end walls, in which at least one inlet channel from the source is oriented in the side wall so as to generate a vortex of liquid within the chamber, a spray outlet channel is formed in a first of the end walls and a bleed outlet channel is formed in a second of the end walls, wherein the spray outlet channel is narrower than the bleed outlet channel. The narrowness of the spray outlet channel is preferably narrow enough so as to generate a high pressure portion of the chamber towards the spray outlet channel end of the chamber. The wider bleed outlet channel is dimensioned so as to form a relatively low pressure portion of the chamber at the bleed outlet end of the chamber, so as to facilitate the projection of excess fluid out of the bleed outlet channel.

The side wall may be cylindrical, and is preferably a circular cylinder (as opposed for example, to a chamber with an elliptical transverse cross-section), and each inlet channel is preferably oriented so to introduce liquid into the chamber in a direction substantially tangential to a portion of the side wall adjacent the inlet channel.

So as to form the spray at the spray outlet channel, the first end wall may taper outwardly from the chamber to an apex where the spray outlet channel is formed.

In a preferred embodiment, the chamber has a longitudinal axis of symmetry and openings from the chamber into the spray outlet channel and into the bleed outlet channel are located substantially co-axially with the longitudinal axis. By using this arrangement, the action of the bleed outlet channel occurs around the central axis of the chamber and so does not effect any vortex formed in the chamber towards the spray outlet channel, as any such vortex is formed adjacent to the side wall of the chamber. So as to facilitate smooth formation of a vortex to the spray outlet channel, the length of the chamber between the end walls is at least twice the width of the chamber between opposing faces of the side wall. Such a relatively long chamber is preferred as it allows the flow of liquid in the vortex to settle down, allows liquid to bleed off out of the bleed outlet channel and separates the spray outlet channel and the bleed outlet channel, sufficiently that the action of the bleed outlet channel has minimal effect on droplet size. Furthermore, this arrangement allows the internal flow to attain symmetry and therefore for the spray device to generate a spray which is substantially symmetrical and of a narrow angle, for example of an angle in the range of from less than about 60°, particularly less than about 40°.

It is preferred that the total cross-sectional area of the inlet channel(s) is at least twice the area of the spray outlet channel. This arrangement aids in the generation of a spray of a narrow angle as discussed above.

The spray outlet channel preferably has a diameter of less than 0.7mm. This diameter of outlet can be reproduced using fairly standard manufacturing techniques, while generating a very desirable fine spray. The droplets in the spray preferably have a mass median drop size of less than 80 microns and more preferably less than 30 microns. The flow rate of the liquid from the spray outlet channel of each atomiser is preferably less than 0.3 litres per minute and more preferably less than 0.1 litres per minute. Also, the pressure of the liquid as it is fed into the, or each atomiser is preferably at least 30 atmospheres (3MPa) and more preferably at least 80 atmospheres (8 MPa).

The spray device may additionally comprise an arrangement for varying the cross-sectional area of the bleed outlet channel so as to vary the spray outlet flow rate. This arrangement can be incorporated in a shut off mechanism (as discussed below) by having a tapered or stepped portion of the spindle adjacent to the opening from the housing into the bleed outlet opening.

The devices as described above comprising an atomiser having a housing with a side wall and two end walls may additionally comprise for the, or each atomiser, a shut off system. The shut off system preferably comprises a rod having a tip shaped to close off the spray outlet opening which is slideably mounted within the chamber so as to extend substantially equidistantly between opposing surfaces of the side wall of the chamber, wherein in a first forward position, the tip of the rod closes off the spray outlet channel, and in a second rearward position, the tip of the rod moves away from the spray outlet opening so as to open the spray outlet opening without disrupting the flow of any such vortex generated within the chamber. When the rod is in its first forward position liquid fed into the atomiser bleeds out of the bleed outlet channel and may be recirculated back to the source. When the rod is in its second rearward position, a proportion of the liquid fed into the atomiser is dispensed through the spray outlet channel as a fine spray and the remainder of the liquid bleeds out of the bleed outlet channel. The rod may be conveniently slideably mounted in the second end wall of the atomiser housing and may be biased by a spring element into the forward position in which the spray outlet channel is closed off. The central location of the rod within the chamber does not damage the vortex of liquid generated in the chamber, as the vortex spirals around the walls of the chamber.

The present invention incorporating the shut off system described above, may be utilised in a hand held spray device, but comprising only a single atomiser. The device may further comprise a housing defining a handle, a forward end and a trigger mount, wherein the atomiser is mounted at the forward end and a trigger arrangement is mounted in the trigger mount such that when the trigger is not actuated the rod is in its first forward position, and when the trigger is actuated the rod moves to its second rearward position and a spray may be dispensed from the forward end of the device.

The present invention may be utilised in a trolley based spray device, for example for decontaminating a room or people located within a room and which comprises a plurality of the atomisers. In this embodiment the atomisers are mounted at an elevated position on a support trolley, which supports the source and a system for recirculating any excess liquid from the atomisers to the source. This device may additionally comprise the hand held device described above, wherein the atomisers of the devices are fed from a common source. Thus the elevated nozzles can be used to disperse a fine decontamination spray through the room and the hand held device can be used to direct a fine decontamination spray in hard to reach locations.

The present invention may also be utilised in a walk-through spray device which may comprise a plurality of the atomisers, wherein the atomisers may be mounted around a door shaped frame, so as to dispense a spray into the space within the frame. Alternatively, the atomisers may be mounted within a cubicle, so as to dispense a spray into the cubicle.

Also, the present invention may be utilised on a hand spray device which may comprise a plurality of the atomisers, wherein the device may have a housing defining a cavity suitable for receiving a pair of hands, and the nozzles may be mounted within the cavity, so as to dispense a spray into the cavity.

The liquid dispensed by the spray devices according to the present invention may be water or an aqueous solution, but other solvents, such as alcohol or ethanol may also be used. Where the spray device is a decontamination device, the liquid contains a decontamination agent.

The spray devices according to the present invention may have a cut off system for the spray outlet channel in which movement of the rod is actuated manually. Alternatively, the rod may be actuated remotely, for example, via an electromechanical, pneumatic or hydraulic actuation system.

The invention will now be described by way of example only and with reference to the accompanying schematic drawings, wherein:
Figure 1A shows a representation of a decontamination device according to the present invention, incorporating a plurality of atomising nozzles;
Figure 1B shows an enlargement of the portion of Figure 1A labelled A;
Figure 2A shows a longitudinal cross-section of a first design of atomising nozzle which can be used in the device of Figure 1;
Figure 2B shows a transverse cross-section through line A-A of Figure 2A;
Figure 3 shows a longitudinal cross-section of a second design of atomising nozzle, incorporating an arrangement for shutting off the nozzle, which can be used in the device of Figure 1;
Figure 4 shows a hand held spray device according to the present invention, which utilises the nozzle design of Figure 3;
Figure 5 shows the hand held spray device of Figure 4 connected to a support trolley and in use;
Figure 6 shows the internal structure of a practical implementation of the decontamination device of Figure 1 in combination with the hand held spray device of Figure 4;
Figure 7 shows the implementation of Figure 6 housed within a housing;
Figures 8A and 8B show further embodiments of decontamination devices incorporating the nozzles of Figure 2 in an arrangement similar to that shown in Figure 1;
Figure 9 shows a hand decontamination device incorporating the nozzles of

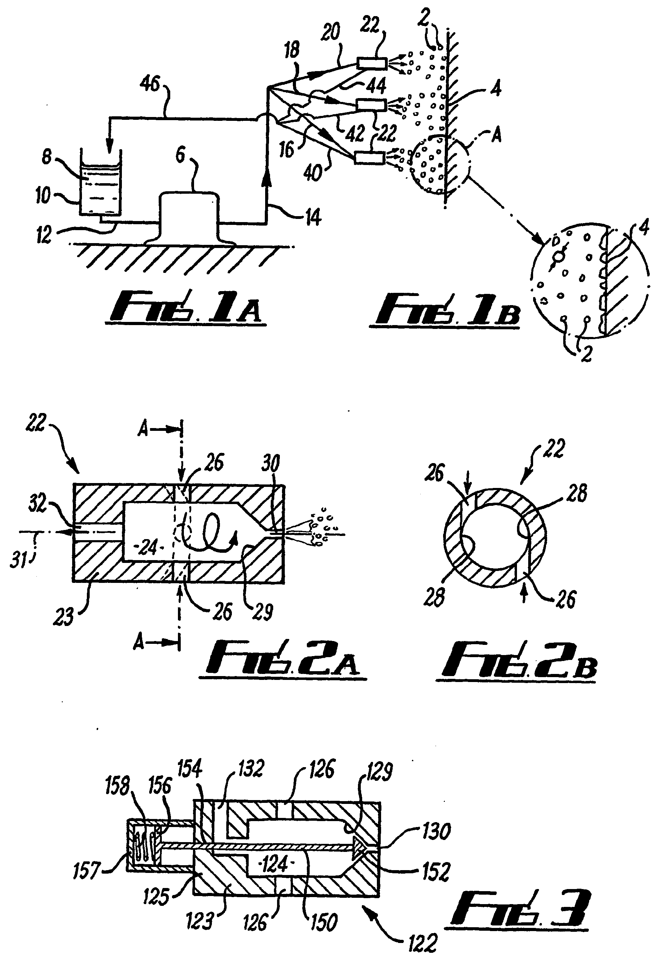

Figure 2 in an arrangement similar to that shown in Figure 1;

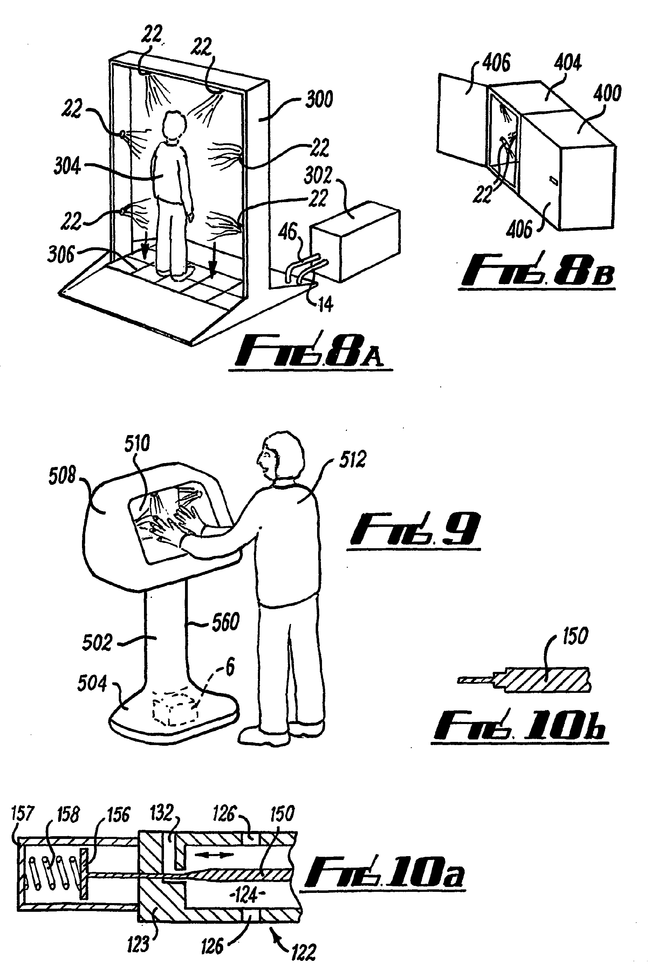

Figure 10a shows the rearward portion of a further embodiment of an atomising nozzle of the type shown in Figure 3 in which the rod or spindle is tapered so as to vary the area from the chamber to the bleed outlet channel;
and
Figure 10b shows an alternative to the tapered portion of the rod or spindle of
Figure 10a, in which to rod or spindle has a stepped profile.
Figures 1A and 1B show a device for dispensing a fine spray or aerosol of liquid droplets (2) onto a surface (4) for antibacterial, disinfectant, decontamination or other hygiene purposes and also for cleaning and cooling purposes. The device comprises a reservoir (10) for storing a liquid (8), out of which the spray is generated. The liquid (8) may include one or more chemically active ingredients which act, for example, as antibacterial, disinfectant or decontamination agents. The device further comprises a pump (6) for pumping the liquid (8) from the reservoir, via first and second supply lines (12, 14), and distribution lines (16, 18, 20) to a plurality of atomising nozzles (22).

Each nozzle (22), as shown in more detail in Figures 2A and 2B, comprises a nozzle housing (23) defining a substantially cylindrical chamber (24). The housing (23) comprises a side wall having a cylindrical internal surface and a pair of end walls. The side wall is formed with one or more tangential inlet channels (26). In the embodiment of Figures 2A and 2B two opposing tangential inlet channels are utilised, which each extend through the side wall of the nozzle housing (23) substantially tangentially to an adjacent portion (28) of the cylindrical internal surface of the side wall of the housing (23). The cylindrical internal surface of the side wall need not be formed as a right cylinder with a circular transverse cross-section, by may also have an oval or elliptical transverse cross-section. Each channel (26) has a substantially circular transverse cross-section, although it is also desirable for the channels to have a substantially rectangular transverse cross-section. The potions (28) of the internal wall of the housing (23) are opposite each other. In Figure 2A, the inlet channels (26) are shown extending at a right angle to the longitudinal axis (31) of the chamber (24), however it is also possible to angle the channels (26), so that they extend away from a spray outlet channel (30), as is shown in dashed lines in Figure 2A.

A stream of the liquid (8) is continuously introduced into the chamber (24), via the two inlet channels (26) under pressure from the pump (6), so that a vortex of liquid is formed within the chamber. As the liquid is injected into the chamber, tangentially and the internal surface of the side wall has a cylindrical internal surface, a vortex of liquid is generated within the chamber, as indicated by the arrow in Figure 2A. The vortex of liquid is located adjacent to the walls of the chamber and moves in a spiral path from the inlets towards a spray outlet (30).

The spray outlet channel (30) is formed through a first end wall of the housing (23) from which the spray is dispensed. The portion of the chamber between the inlet openings (26) and the spray outlet (30) is a high pressure portion of the chamber (24) due to the small diameter of the spray outlet (30). The first end wall is formed with a conical internal surface (29), which tapers to an apex in a direction away from the centre of the chamber (24), and the spray outlet channel (30) is formed at the apex of the conical surface (29). This conical surface directs the vortex of liquid of the spray outlet (30)

A bleed outlet channel (32) is formed through a second end wall of the housing (23) at the opposite end of the housing to the first end wall. The bleed outlet channel (32) bleeds excess liquid from the chamber (24), so that this excess liquid is not atomised, but instead is recirculated to the reservoir (10) via a set of return lines (40-46). The bleed outlet (32) has a larger diameter than the spray outlet (30) and so that portion of the chamber between the inlet openings (26) and the bleed outlet is a lower pressure portion of the chamber (24). A vortex of liquid extending from the inlet openings (26) to the bleed outlet (32) is also formed which acts to squirt excess liquid into the return lines (40-46) and back to the reservoir (10) for recirculation. The bleed outlet channel (32) is located in a low pressure region of the chamber (24), opposite to the spray outlet channel (30) such that the presence of the bleed opening (32) does not disrupt the angular momentum of the liquid (8) in the vortex to the spray outlet (30).

The opening from the chamber (24) into the bleed outlet channel (32) and the opening from the chamber (24) into the spray outlet chamber (30) are co-axial and lie on the longitudinal axis of symmetry (31) of the chamber (24). The co-axial location of the bleed outlet channel (32) prevents the bleeding of excess fluid through the channel from disrupting the vortex of liquid to the spray outlet channel (30).

The droplets (2) are produced by energy supplied to the liquid (8) by the pump (6). The pump (6) supplies liquid (8) to the inlet nozzles (26) at sufficiently high pressure for a fine spray dispersion to be dispensed from the spray outlet channel (30) so as to provide an even coating of droplets (2) for the surface (8), without excessive liquid deposition. The pressure generated by the pump (6), as measured at the interface between the feed lines (16, 18, 20) and the inlet channels (26) is preferably not less than 30 atmospheres (3MPa), and more preferably not less than 80 atmospheres (8MPa). This generates a mass median drop size of preferably not more than 80 microns and more preferably not more than 30 microns.

The bleed outlet channel (32) is required in order to generate the required small droplet size at the required low liquid flow rate, while maintaining a spray outlet nozzle diameter, of less than 0.7mm and preferably less than 0.3mm, which can be generated using mass production techniques. The proportion of the liquid (8) fed into the chamber (24) which is bled off via the bleed outlet channel (32) can be determined by setting the transverse cross-sectional area of the bleed outlet channel (32), or by generating a restriction in the flow of liquid along the bleed outlet channel (32) or the return lines (40-46). In a preferred embodiment, the exit orifice to the spray outlet channel (30) is no less than 0.2mm in diameter and the spray flow rate is not more than 0.3 litres per minute, with at least half of the liquid (8) fed into the chamber (24) being bled off via the bleed outlet channel (32) and recirculated to the reservoir (10).

As an alternative to the spray nozzle (22) shown in Figures 1A, 2A and 2B, the design of nozzle (122) shown in Figure 3 can be used. The nozzle (122) of Figure 3, has a housing (123) defining a chamber (124) of a shape, with tangential inlet channels and with a spray outlet channel (130) similar to those of Figures 2A and 2B. The bleed outlet channel (132), is also similar to that shown in Figures 2A and 2B, except that the channel (132) is formed with a 90° bend in it. Thus, the spray nozzle (122) operates in the same way as the spray nozzle (22), except that the spray nozzle (122) can be shut off by a shut off mechanism.

The shut off mechanism, comprises a spindle (150) which extends co-axially along the longitudinal axis of the chamber (124) and co-axial with the spray outlet channel (130) and the opening from the bleed outlet channel (132) into the chamber (124). A forward end of the spindle (150) has a conical tip (152) which is dimensioned to mate with the portion of conical end wall (129) surrounding the entrance of the spray outlet channel (130) so as to block the spray outlet channel. The spindle (150) is slideably mounted within a spindle channel (154), which extends rearwardly from the 90° bend in the bleed outlet channel (132) through the housing (123), so that a rearward end of the spindle (150) extends beyond the external surface of the second end wall (125) of the housing (123). The rearward end of the spindle (150) is terminated with a stop plate (156). A spring (158) is positioned behind the stop plate (156), extending from the surface of the stop plate (156) facing away from the second end wall (125) and a spring support (157). The spring support (157) is rigidly fitted to the housing (122) and acts to support the spring (158), so as to bias the spindle (150) forwardly so that the tip (152) of the spindle (150) blocks the opening to the spray outlet channel (130). A force can be applied to the stop plate (156) against the biasing force of the spring (158) so as to move the spindle (150) rearwardly, into the position shown in Figure 3, in which the tip (152) of the spindle (150) is withdrawn from the opening of the spray outlet channel (130), so that the spray outlet channel (130) is opened. This form of shut off mechanism, with a spindle (150) which extends co-axially with the chamber (124) enables the spray from the spray outlet channel (130) to be shut off, as required, without disrupting the vortex of liquid within the chamber (124). The tip (152) is sufficiently small and is withdrawn from the spray outlet channel (30) by a sufficient distance so that when it is withdrawn into the position shown in Figure 3, the presence of the tip (152) does not disrupt the flow of the vortex of liquid towards the spray outlet channel (30).

With the shut off mechanism in the open position shown in Figure 3, the spray outlet channel (130) is open and the nozzle (122) operates in a similar way to the nozzle (22) described above in relation to Figures 2A and 2B. However, with the shut off mechanism on a closed position in which the tip (152) of the spindle (150) blocks the spray outlet channel (130), all the liquid entering the chamber (124) via the inlets (126) is bled though the bleed outlet channel (132) and recirculated back to the reservoir (10). In this way, the shut off mechanism can be used to shut off, the spray nozzles (122).

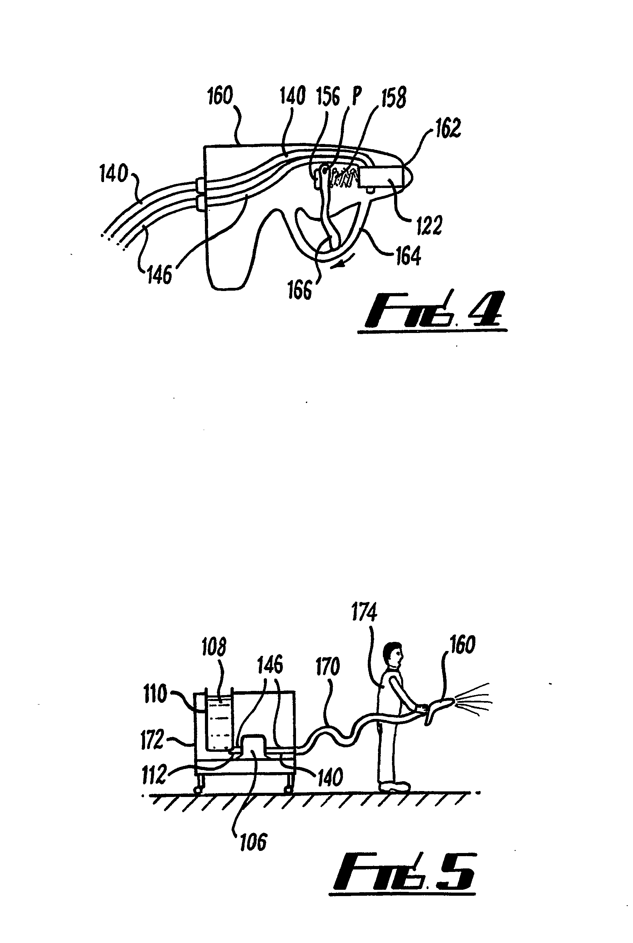

The nozzle design of Figure 3 can be utilised in a hand held spray device, of the type shown in Figures 4 and 5. The spray device has a housing (160), which may be a clamshell type housing, shown with the top half of the clamshell removed in Figure 4. The device comprises a single spray nozzle (122), mounted within the housing with the spray outlet channel end of the nozzle extending from a front outlet opening (162) of the housing. The nozzle (122) has a pair of tangential inlet channels, into which liquid is fed via supply line (140). The supply line (140) extends from the spray nozzle (122) through the housing (160) and out of the rear of the housing to a pump supply (see Figure 5), comprising a pump (106) and a reservoir (110) which operate in similar way to that shown in Figure 1. The nozzle (122) has a bleed outlet channel which leads into a return line (146). The return line (146) extends from the spray nozzle (122) through the housing (160) and out of the rear of the housing to the reservoir (110). The housing defines a handle (164), suitable to be gripped by a user of the hand held device, and a trigger mount, within which is pivotally mounted a trigger (166) so as to pivot about point (P). With the trigger not depressed, the shut off mechanism (156, 158) of the nozzle (122) blocks the spray outlet channel (130) of the nozzle. When a lower end of the trigger (166), which extends out of the housing (160) is depressed in the direction of the arrow in Figure 4, a portion of the trigger (166) bears against the stop plate (156) of the shut off mechanism. The portion of the trigger (166) acts on the stop plate (156) to pull the stop plate (156) and thus the spindle (150) rearwardly, against the biasing force of the spring (158), into the position shown in Figure 3, so as to open the nozzle (122). When the nozzle (122) is open a proportion of the liquid fed to it via the supply line (140) is dispensed from the spray outlet channel (130) of the nozzle in a fine spray, while the remainder of the liquid is bled from the nozzle via the bleed outlet channel (132).

Figure 4 shows hand actuation of the shut off mechanism, although alternatively, the shut off mechanism could be actuated in other ways. One alternative would be to actuate the shut off mechanism using and electromechanical system, for example using a solenoid based actuation system. Other alternatives include pneumatic or hydraulic actuation systems.

As can be seen in Figure 5, the supply line (140) and return line (146) can be held side by side within a tubular cover (170) which extends from the housing (160) of the hand held spray device to a support trolley (172). At the support trolley (172), the supply line (140) extends from the tubular cover to a pump (106) supported in the support trolley. Similarly, at the support trolley (172), the return line (146) extends from the tubular cover to a reservoir (110). A feed line (112) extends from the reservoir (110) to the pump (106). To use the hand held spray device, a user (174) switches on the pump (106) which causes liquid (108) to be drawn from the reservoir (110) and to be circulated from the pump (106), along feed line (140) to the nozzle (122) and from the nozzle (122) along the return line (146) to the reservoir (110). Then when the user depresses the trigger (166) of the device, a proportion of the liquid (108) supplied to the nozzle (122) is dispensed from the spray outlet channel (130) of the nozzle in a fine spray, and the remainder of the liquid is bled from the nozzle via the bleed outlet channel (132) and circulated back to the reservoir (110). The rearward end of an alternative design of shut off mechanism to that shown in Figure 3 is shown in Figure 10a, with like parts identified with like numerals. In the Figure 10a embodiment the spindle (150) is tapered so that movement of the spindle in the direction of the arrow will vary the area of the outlet from the chamber (124) into the bleed outlet channel (132). By varying this area, the spray flow rate through the spray outlet channel (130) can be varied. As an alternative to tapering the spindle as shown in Figure 10a, the spindle could be stepped as shown in Figure 10b. In Figures 10a and 10b, the stepped or tapered portion of the spindle (150) of the cut off mechanism is used to vary the area of the outlet from the chamber (124) into the bleed outlet channel, however, the arrangement for varying the area of this outlet need not be part of the shut off mechanism, but could instead be an independent arrangement. Also, such an independent arrangement could be located anywhere in the bleed outlet channel (132) to vary the cross-sectional area of that channel so as to vary the flow rate at the spray outlet channel.

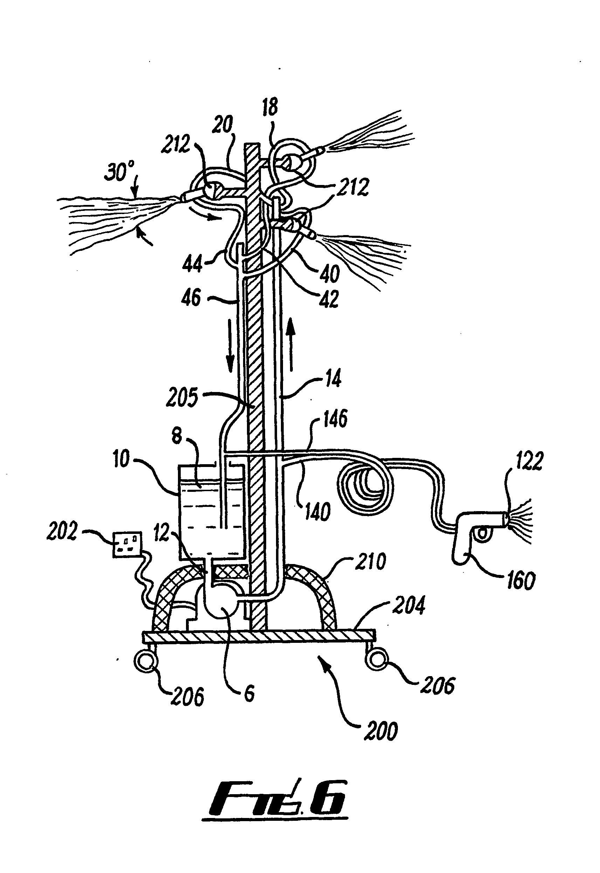

Figure 6 shows the decontamination device of Figure 1 and the spray gun of Figure 4, mounted on a support trolley (200), with like parts of Figures 1, 4 and 6 identified by like numerals. The support trolley (200) comprises a base (204) supported on casters (206), so that the trolley can be easily moved around. A central support pole (205) extends upwardly from the base (204).

A five litre polymer reservoir (10) is mounted on the support trolley (200) and is at atmospheric pressure, making it easy to refill. The reservoir (10) contains a decontamination liquid (8), for example a solvent, such as water or ethanol, in which a decontaminant solute is dissolved. A supply line (12) extends from base of the reservoir (10) to a pump (6), which is driven by an electric motor (not shown), powered by a mains electricity supply (202). The pump (6) is mounted on the base (204) of the support trolley (200) and is surrounded by a layer of soundproofing (210), mounted on the base (204). The layer of sound proofing (210) forms a covering for the pump (6) through which the supply lines (12, 14) and the central support pole (205) extend.

The pump (6) pumps the liquid along supply lines (14, 16, 18, 20) to three spray nozzles (22) of the type shown in Figure 2. The supply lines (14 to 20) may, for example, be 6mm bore stainless steel pipes. Each nozzle (22) is mounted via an associated ball and socket joint (212) to the central support pole (205). The nozzles (22) are mounted at an elevated position above the level of the reservoir (10) so that gravity aids in the return of liquid from the nozzles (22) to the reservoir (10). The ball and socket joints (212) enable the spray nozzles (22) to be directed in a desired direction. Each nozzle (22) has a 10mm outer diameter and a length of 40mm and is designed to generate a stream of spray, which exits the nozzle to provide spray coverage over a 30° angle. The liquid leaving the nozzles (22) via the bleed openings (32) is fed back to the reservoir (10) via return lines (40, 42, 44, 46), which are at atmospheric pressure and may for example comprise 10mm diameter stainless steel pipes.

The supply line from the pump (6) branches off into a supply line (140) of a hand-held spray device of the type shown in Figure 4, having housing (160). Any liquid leaving the bleed opening (132) of the nozzle (122) of the spray device is returned to the reservoir via return line (146).

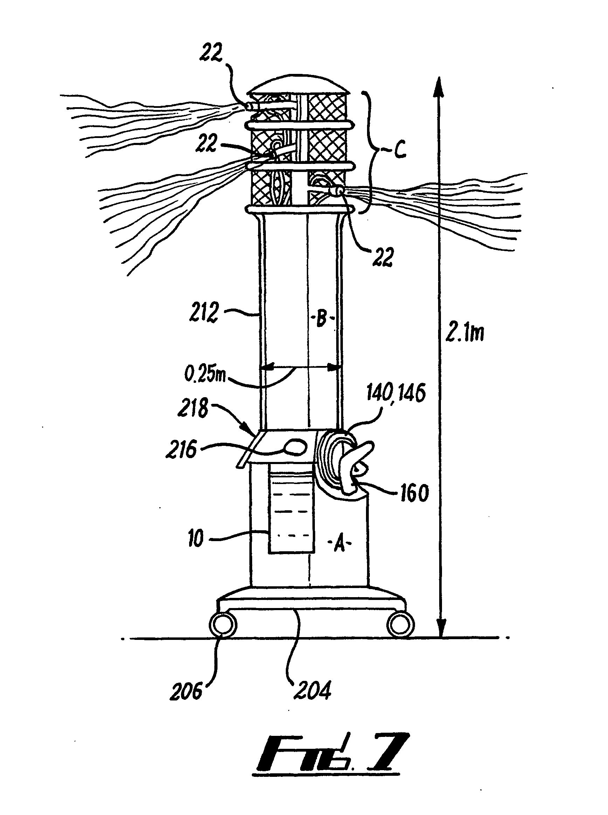

The workings of the spray device shown in Figure 6 are preferably covered by a housing (212), as is shown in Figure 7. The housing (212) is mounted on the base (204) of the support trolley (200) and is shaped to house the pump (6), reservoir (10), central pole (205), pipe work (12, 14, 16, 18, 20) and (40, 42, 44, 46) and nozzles (22). A widened base (A) of the housing (212) is formed with a recess or a clear panel, through which can be seen the liquid level in the reservoir (10), an openable hatch (216) through which the reservoir (10) can be refilled and a recess for stowing the hand held spray device (160) and its supply and return lines (140, 146) (see Figure 4). A control panel (218) is mounted on the housing (212), via which the pump (6) can be switched on and off. A cylindrical central portion (B) of the housing (212) extends upwardly from the widened base portion (A) and terminates in a three tiered turret portion (C). The turret portion (C) has three vertically spaced openings, out of which a respective one of the nozzles (22) extends. The central portion (B) of the housing may have a horizontal diameter of around 0.25m and the housing (214) may extend to a height of around 2.1m.

The spray device of Figures 6 and 7 can be located in an enclosed space, for example a room, for decontaminating a room, or for decontaminating people in the room. The nozzles (22) generate a fine spray which is distributed around the room and the hand held spray device (160) can be used for decontaminating any areas, which are protected from the spray generated by the nozzles, for example, which are located under or behind objects.

Substantially, the same device can be constructed in order to cool the environment by the evaporative action of fine sprays of water or alternatively, to clean the atmosphere of particles, such as particles of tobacco smoke.

Figure 8A shows a walk through decontamination device, comprising a door shaped support housing (300), within which are mounted a plurality of spray nozzles (22). The spray nozzles are fed with a decontaminating liquid from a reservoir contained in a support unit (302), which unit also contains a pump for pumping the liquid from the reservoir. Liquid is pumped from the reservoir in the support unit (302) via the supply line (14) into a network of supply lines, housed within the door shaped support housing (300), to the nozzles (22). The liquid bled from the nozzles (22) is fed back to the reservoir in the support unit (302) via a network of return lines, including return line (146). A person to be decontaminated (304), simply stands within the support housing (300). Any excess liquid which it dispensed through the nozzles (22) in a spray is collected in the base of the support housing (302), which base comprises a collection reservoir covered by a mesh (306) on which the person (304) stands. In an alternative embodiment to that shown in Figure 8A, a row of cubicles (400, 404) can be provided, as shown in Figure 8B each containing a plurality of nozzles (22) and fed by liquid from a reservoir via a pump, as described above. A person to be contaminated, simple opens the door (406) to the cubicle and walks inside, closing the door behind them and stands in the spray from the nozzles (22) for a predetermined period of time.

Figure 9 shows a hand decontamination device according to a further embodiment of the present invention. The device comprises a housing (502), comprising a base (504), which houses a pump (6) and a reservoir (not shown) storing a decontamination liquid, in a similar way to the base (A) of the housing (212) of Figure 7. Extending upwardly from the base is a central column (560) of the housing, which houses a central support pole and pipe work similar to the housing portion (B) of the housing (212) of Figure 7. The central column (560) terminates in a housing portion (508), which defines a cavity (510), at a suitable height above the ground and of a suitable size for a person to place their hands within the cavity. The cavity (510) houses a plurality of nozzles (22) for dispensing a decontaminating spray as is described above in relation to Figure 2. The device of Figure 9 may for example be used in a hospital for removal of bacteria from hands, for example as a member of staff moves between different patients. The person (512) simply places there hands within the cavity (510) and activates a switch (which may be located within the cavity) for starting the pump in the base (504). Then they place their hands in the cavity (510) for a predetermined period of time, during which their hands are sprayed from the nozzles (22) by a decontaminating liquid. The person may then deactivate the pump by switching the switch or a timing device may be included for deactivating the pump after the predetermined time period.

## Claims

1. A spray device comprising at least one swirl atomiser (22) for generating a spray of a liquid and a source of pressurised liquid for feeding the or each swirl atomiser, wherein each swirl atomiser comprises:
a housing (23) defining a tubular chamber (24) having a side wall and two opposing end walls;
at least one inlet channel (26) from the source oriented in the side wall so as to generate a vortex of liquid within the chamber (24);
a bleed outlet channel (32) in a second of the end walls for bleeding any excess liquid from the atomiser (22); and
a spray outlet channel (30) in a first of the end walls, which is narrower than the bleed outlet channel (32);
**characterised in** the length of the chamber between the end walls Is at least twice the width of the chamber (24) between opposing faces of the side wall.

2. A device according to claim 1, additionally comprising a recirculation system for recirculating the excess liquid to the source.

3. A device according to any one of the preceding claims, wherein the source of pressurised liquid comprises a reservoir of liquid (10) and a pump (6) for pumping liquid from the reservoir (10) to the or each atomiser (22).

4. A device according to claim 1, wherein the side wall is cylindrical and each inlet channel (126) is oriented so to introduce liquid into the chamber (124) in a direction substantially tangential to a portion of the side wall adjacent the inlet channel (126).

5. A device according to any one of the preceding claims, wherein the total cross-sectional area of the inlet channel(s) (26) is at least twice the area of the spray outlet channel (30).

6. A device according to any one of the preceding claims, wherein the spray outlet channel (30) has a diameter of less than 0.7mm.

7. A device according to any one of the preceding claims for generating droplets having a mass median drop size of less than 80 microns

8. A device according to any one of the preceding claims, wherein the device is adapted to provide a flow rate of the liquid from the spray outlet Channel (30) of each atomiser of less than 0.3 litres per minute.

9. A device according to any one of the preceding claims, wherein the device is adapted to provide a pressure of the liquid as it is fed into the or each atomiser of at least 30 atmospheres (3MPa).

10. A device according to any one of the preceding claims additionally comprising for the, or each atomiser (22), a shut off system, comprising:
a rod (150) having a tip (152) shaped to close off a spray outlet opening (130) which is slideably mounted within the chamber (124) so as to extend substantially equidistantly between opposing surfaces of the side wall of the chamber;
wherein in a first forward position, the tip (152) of the rod (150) is arranged to close off the spray outlet channel (130), and in a second rearward position, the tip (152) of the rod (150) is located away from the spray outlet opening (130) so as to open the spray outlet opening (130) without disrupting the flow of any such vortex generated within the chamber (124).

11. A device according to claim 10, wherein the rod (150) biasable is by a spring element (158) into the forward position in which the spray outlet channel (130) is closed off.

12. A spray device according to claim 10 or 11 comprising a single atomiser (122) and a housing (160) defining a handle (164), a forward end and a trigger mount, wherein the atomiser (122) is mounted at the forward end and a trigger arrangement (166) is mounted in the trigger mount such that:
when the trigger arrangement (166) is not actuated the rod (152) is in its first forward position, and when the trigger arrangement (166) is actuated the rod (152) is in its second rearward position whereby the spray is dispensed from the forward end of the device.

13. A spray device according to any one of claims 10 to 12, wherein the movement of the rod (152) is manually actuatable.

14. A spray device according to claim 10 or 11, wherein the movement of the rod (152) is arranged to be actuated remotely via an electromechanical, pneumatic or hydraulic actuation system.

15. A spray device according to any one of claims 1 to 11 and 14, comprising a plurality of the atomisers (22), wherein the atomisers are mounted at an elevated position on a support trolley (204), which support trolley supports the source (10) and a recirculation system for recirculating the excess liquid from the atomisers (22) to the source (10).

16. A device according to any one of claims 1 to 11 and 14, comprising a plurality of the atomisers (22), wherein the atomisers (22) are mounted around a door shaped frame (300), so as to dispense the spray into the space within the frame.

17. A spray device according to any one of claims 1 to 11 and 14, comprising a plurality of the atomisers (22), wherein the atomisers are mounted within a cubicle (404), so as to dispense the spray into the cubicle.

18. A spray device according to any one of claims 1 to 11 and 14, comprising a plurality of the atomisers (22), wherein the device has a housing (508) defining a cavity (510) suitable for receiving a pair of hands, and the atomisers (22) are mounted within the cavity (510), so as to dispense the spray into the cavity (510).

19. A spray device according to any one of the preceding claims additionally comprising an arrangement for varying the cross-sectional area of the bleed outlet channel (32) whereby the spray outlet flow rate is varied.

20. A spray device according to any one of claims 1 to 19 used for decontamination or for the cleaning of surfaces, wherein the liquid comprises a decontamination agent or a detergent respectively, or used for cleaning or cooling of the atmosphere, wherein the liquid comprises water.

## Patentansprüche

1. Sprühvorrichtung, die mindestens einen Wirbelzerstäuber (22) für das Erzeugen eines Flüssigkeitssprays und eine Druckflüssigkeitsquelle für das Versorgen des oder eines jeden Wirbelzerstäubers aufweist, wobei jeder Wirbelzerstäuber aufweist:
ein Gehäuse (23), das eine rohrförmige Kammer (24) mit einer Seitenwand und zwei entgegengesetzten Stirnwänden aufweist;
mindestens einen Einlasskanal (26) von der Quelle, ausgerichtet in der Seitenwand, um so einen Flüssigkeitswirbel innerhalb der Kammer (24) zu erzeugen;
einen Ablassaustrittskanal (32) in einer zweiten der Stirnwände für das Ablassen jeglicher überschüssiger Flüssigkeit aus dem Zerstäuber (22); und
einen Sprayaustrittskanal (30) in einer ersten der Stirnwände, der schmaler ist als der Ablassaustrittskanal (32),
**dadurch gekennzeichnet, dass** die Länge der Kammer zwischen den Stirnwänden mindestens das 2-fache der Breite der Kammer (24) zwischen den entgegengesetzten Oberflächen der Seitenwand beträgt.

2. Vorrichtung nach Anspruch 1, die zusätzlich ein Rückführungssystem für das Rückführen der überschüssigen Flüssigkeit zur Quelle aufweist.

3. Vorrichtung nach einem der vorhergehenden Ansprüche, bei der die Druckflüssigkeitsquelle einen Flüssigkeitsbehälter (10) und eine Pumpe (6) für das Pumpen der Flüssigkeit vom Behälter (10) zu dem oder jedem Zerstäuber (22) aufweist.

4. Vorrichtung nach Anspruch 1, bei der die Seitenwand zylindrisch ist und jeder Einlasskanal (126) ausgerichtet ist, um so Flüssigkeit in die Kammer (124) in einer Richtung im Wesentlichen tangential zu einem Abschnitt der Seitenwand benachbart dem Einlasskanal (126) einzuführen.

5. Vorrichtung nach einem der vorhergehenden Ansprüche, bei der die gesamte Querschnittsfläche des (der) Einlasskanals (Einlasskanäle) (26) mindestens das 2-fache der Fläche des Sprayaustrittskanals (30) beträgt.

6. Vorrichtung nach einem der vorhergehenden Ansprüche, bei der der Sprayaustrittskanal (30) einen Durchmesser von weniger als 0,7 mm aufweist.

7. Vorrichtung nach einem der vorhergehenden Ansprüche für das Erzeugen von Tröpfchen mit einer massebezogenen mittleren Tropfengröße von weniger als 80 µm.

8. Vorrichtung nach einem der vorhergehenden Ansprüche, bei der die Vorrichtung so ausgebildet ist, dass sie eine Strömungsgeschwindigkeit der Flüssigkeit aus dem Sprayaustrittskanal (30) eines jeden Zerstäubers von weniger als 0,3 Liter pro Minute liefert.

9. Vorrichtung nach einem der vorhergehenden Ansprüche, bei der die Vorrichtung so ausgebildet ist, dass sie einen Druck der Flüssigkeit, während sie in den oder jeden Zerstäuber zugeführt wird, von mindestens 30 Atmosphären (3 MPa) liefert.

10. Vorrichtung nach einem der vorhergehenden Ansprüche, die zusätzlich für den oder jeden Zerstäuber (22) ein Absperrsystem aufweist, das aufweist:
eine Stange (150) mit einem Ende (152), das so geformt ist, dass es eine Sprayaustrittsöffhung (130) verschließt, die verschiebbar innerhalb der Kammer (124) montiert ist, um sich im Wesentlichen abstandsgleich zwischen entgegengesetzten Flächen der Seitenwand der Kammer zu erstrecken;
wobei in einer ersten vorderen Position das Ende (152) der Stange dazu ausgebildet ist, den Sprayaustrittskanal (130) zu verschließen, und wobei in einer zweiten hinteren Position das Ende (152) der Stange (150) weg von der Sprayaustrittsöffnung (130) angeordnet ist, um so die Sprayaustrittsöffnung (130) zu öffnen, ohne dass der Strom irgendeines derartigen innerhalb der Kammer (124) erzeugten Wirbels unterbrochen wird.

11. Vorrichtung nach Anspruch 10, bei der die Stange (150) mittels eines Federelementes (158) in die vordere Position vorgespannt werden kann, in der der Sprayaustrittskanal (130) verschlossen ist.

12. Sprühvorrichtung nach Anspruch 10 oder 11, die einen einzelnen Zerstäuber (122) und ein Gehäuse (160) aufweist, das einen Griff (164), ein vorderes Ende und eine Abzugshalterung definiert wobei der Zerstäuber (122) am vorderen Ende montiert ist, und wobei eine Abzugsanordnung (166) in der Abzugshalterung montiert ist, so dass:
wenn die Abzugsanordnung (166) nicht betätigt wird, die Stange (152) in ihrer ersten vorderen Position ist, und, wenn die Abzugsanordnung (166) betätigt wird, die Stange (152) in ihrer zweiten hinteren Position ist, wobei das Spray aus dem vorderen Ende der Vorrichtung ausgegeben wird.

13. Sprühvorrichtung nach einem der Ansprüche 10 bis 12, bei der die Bewegung der Stange (152) manuell betätigt werden kann.

14. Sprühvorrichtung nach Anspruch 10 oder 11, bei der die Bewegung der Stange (152) dazu gedacht ist, dass sie mittels eines elektromechanischen, pneumatischen oder hydraulischen Betätigungssystems fernbetätigt wird.

15. Sprühvorrichtung nach einem der Ansprüche 1 bis 11 und 14, die eine Vielzahl von Zerstäubern (22) aufweist, wobei die Zerstäuber in einer erhöhten Position auf einem Trägerwagen (204) montiert sind, wobei der Trägerwagen die Quelle (10) und ein Rückführungssystem für das Rückführen der überschüssigen Flüssigkeit von den Zerstäubern (22) zur Quelle (10) trägt.

16. Vorrichtung nach einem der Ansprüche 1 bis 11 und 14, die eine Vielzahl von Zerstäubern (22) aufweist, wobei die Zerstäuber (22) um einen türartigen Rahmen (300) montiert sind, um so das Spray im Raum innerhalb des Rahmens zu verteilen.

17. Sprühvorrichtung nach einem der Ansprüche 1 bis 11 und 14, die eine Vielzahl von Zerstäubern (22) aufweist, wobei die Zerstäuber innerhalb einer Kabine (404) montiert sind, um so das Spray in der Kabine zu verteilten.

18. Sprühvorrichtung nach einem der Ansprüche 1 bis 11 und 14, die eine Vielzahl von Zerstäubern (22) aufweist, wobei die Vorrichtung ein Gehäuse (508) aufweist, das einen Hohlraum (510) definiert, der für das Aufnehmen eines Paares von Händen geeignet ist, und wobei die Zerstäuber (22) innerhalb des Hohlraumes (510) montiert sind, um so das Spray im Hohlraum (510) zu verteilen.

19. Sprühvorrichtung nach einem der vorhergehenden Ansprüche, die zusätzlich eine Anordnung für das Verändern der Querschnittsfläche des Ablassaustrittskanals (32) aufweist, wodurch die Strömungsgeschwindigkeit des Sprayaustrittes verändert wird.

20. Sprühvorrichtung nach einem der Ansprüche 1 bis 19, die für die Dekontaminierung oder für die Reinigung von Oberflächen eingesetzt wird, wobei die Flüssigkeit jeweils ein Dekontaminierungsmittel oder ein Reinigungsmittel aufweist, oder die für die Reinigung oder Abkühlung der Atmosphäre eingesetzt wird, wobei die Flüssigkeit Wasser aufweist.

## Revendications

1. Dispositif de pulvérisation, comprenant au moins un atomiseur à turbulence (23) pour entraîner une pulvérisation d'un liquide, et une source de liquide sous pression, pour alimenter le ou chaque atomiseur à turbulence, chaque atomiseur à turbulence comprenant :
un boîtier (29), définissait une chambre tubulaire (24) comportant une paroi latérale et deux parois d'extrémité opposées ;
au moins un canal d'entrée (26) à partir de la source, orienté dans la paroi latérale de sorte à produire un tourbillon de liquide dans la chambre (24) ;
un canal de sortie de purge (32) dans une deuxième paroi des parois d'extrémité, pour purger un quelconque liquide excédentaire de l'atomiseur (22) ; et
un canal de sortie de pulvérisation (30) dans une première paroi des parois d'extrémité, plus étroit que le canal de sortie de purge (32) ;
**caractérisé en ce que** la longueur de la chambre entre les parois d'extrémité représente au moins le double de la largeur de la chambre (24) entre les faces opposées de la paroi latérale.

2. Dispositif selon la revendication 1, comprenant en outre un système de recirculation pour faire recirculer le liquide excédentaire vers la source.

3. Dispositif selon l'une quelconque des revendications précédentes, dans lequel la source de liquide sous pression comprend un réservoir de liquide (10) et une pompe (6) pour pomper le liquide du réservoir (10) vers le ou chaque atomiseur (22).

4. Dispositif selon la revendication 1, dans lequel la paroi latérale est cylindrique, chaque canal d'entrée (126) étant orienté de sorte à introduire le liquide dans la chambre (124) dans une direction pratiquement tangentielle à une partie de la paroi latérale adjacente au canal d'entrée (126).

5. Dispositif selon l'une quelconque des revendications précédentes, dans lequel la surface de section transversale totale du canal (des canaux) d'entrée (26) représente au moins le double de la surface du canal de sortie de pulvérisation (30).

6. Dispositif selon l'une quelconque des revendications précédentes, dans lequel le canal de sortie de pulvérisation (30) a un diamètre inférieur à 0,7 mm.

7. Dispositif selon l'une quelconque des revendications précédentes, destiné à produire des gouttelettes ayant une taille de goutte moyenne en masse inférieure à 80 microns.

8. Dispositif selon l'une quelconque des revendications précédentes, dans lequel le dispositif est adapté pour assurer un débit d'écoulement du liquide à partir du canal de sortie de pulvérisation (30) de chaque atomiseur inférieur à 0,3 litre par minute.

9. Dispositif selon l'une quelconque des revendications précédentes, dans lequel le dispositif est adapté pour établir une pression du liquide, lors de son amenée vers le ou chaque atomiseur, d'au moins 30 atmosphères (3MPa).

10. Dispositif selon l'une quelconque des revendications précédentes, comprenant en outre, pour le ou chaque atomiseur (22), un système de fermeture, comprenant :
une tige (150), comportant une pointe (152) formée de sorte à fermer une ouverture de sortie de pulvérisation (130), montée de manière coulissante dans la chambre (124), de sorte à s'étendre à une distance pratiquement égale entre les surfaces opposées de la paroi latérale de la chambre ;
dans lequel, dans une première position située vers l'avant, la pointe (152) de la tige est agencée de sorte à fermer le canal de sortie de pulvérisation (130), et dans une deuxième position située vers l'arrière, la pointe (152) de la tige (150) est agencée à l'écart de l'ouverture de sortie de pulvérisation (130), de sorte à ouvrir l'ouverture de sortie de pulvérisation (130) sans perturber l'écoulement d'un quelconque tourbillon produit dans la chambre (124).

11. Dispositif selon la revendication 10, dans lequel la tige (150) peut être poussée par un élément de ressort (158) dans la position située vers l'avant, dans laquelle le canal de sortie de pulvérisation (130) est fermé.

12. Dispositif de pulvérisation selon les revendications 10 ou 11, comprenant un seul atomiseur (122) et un boîtier (160) définissant une poignée (164), une extrémité avant et un support de gâchette, l'atomiseur (122) étant monté au niveau de l'extrémité avant et un dispositif de gâchette (166) étant monté dans le support de gâchette, de sorte que :
lorsque le dispositif de gâchette (166) n'est pas actionné, la tige (152) se trouve dans sa première position située vers l'avant, et lorsque le dispositif de gâchette (166) est actionné, la tige (152) se trouve dans sa deuxième position située vers l'arrière, la pulvérisation étant ainsi distribuée à partir de l'extrémité avant du dispositif.

13. Dispositif de pulvérisation selon l'une quelconque des revendications 10 à 12, dans lequel le déplacement de la tige (152) peut être actionné manuellement.

14. Dispositif de pulvérisation selon les revendications 10 ou 11, dans lequel le déplacement de la tige (152) est actionné à distance par l'intermédiaire d'un système d'actionnement électromécanique, pneumatique ou hydraulique.

15. Dispositif de pulvérisation selon l'une quelconque des revendications 1 à 11 et 14, comprenant plusieurs atomiseurs (22), les atomiseurs étant montés au niveau d'une position surélevée sur un chariot de support (204), ce chariot de support supportant la source (10) et un système de recirculation, pour faire recirculer le liquide excédentaire à partir des atomiseurs (22) vers la source (10).

16. Dispositif selon l'une quelconque des revendications 1 à 11 et 14, comprenant plusieurs atomiseurs (22), les atomiseurs (22) étant montés autour d'un cadre en forme de porte (300), de sorte à distribuer la pulvérisation dans l'espace à l'intérieur du cadre.

17. Dispositif de pulvérisation selon l'une quelconque des revendications 1 à 11 et 14, comprenant plusieurs atomiseurs (22), les atomiseurs étant montés dans une cabine (404), de sorte à distribuer la pulvérisation dans la cabine.

18. Dispositif de pulvérisation selon l'une quelconque des revendications 1 à 11 et 14, comprenant plusieurs atomiseurs (22), le dispositif comportant un boîtier (508) définissant une cavité (510) appropriée pour recevoir une paire de mains, les atomiseurs (22) étant montés dans la cavité (510) de sorte à distribuer la pulvérisation dans la cavité (510).

19. Dispositif de pulvérisation selon l'une quelconque des revendications précédentes, comprenant en outre un agencement pour changer la surface de section transversale du canal de sortie de purge (32), pour changer ainsi le débit d'écoulement de sortie de la pulvérisation.

20. Dispositif de pulvérisation selon l'une quelconque des revendications 1 à 19, utilisé pour la décontamination ou le nettoyage de surfaces, le liquide comprenant respectivement un agent de décontamination ou un détergent, ou utilisé pour le nettoyage ou le refroidissement de l'atmosphère, le liquide comprenant de l'eau.
